# EUROPEAN PATENT APPLICATION

(11) **EP 1 859 785 A1**
(43) Date of publication of application: **28.11.2007**
(21) Application number: 07108588.0
(22) Date of filing: 22.05.2007
(51) Int. Cl.: A61K 8/92, A61K 8/97, A61Q 9/04

(54) **Depilatory product based on vegetable oils and use of vegetable oils for depilatory products**

(30) Priority: 22.05.2006 IT MI20061005
(71) Applicant: R.I.C.A S.p.A., 95040 Motta Sant'Anastasia (CT) (IT)
(72) Inventor: Puglisi, Nunzio, 95126, Catania (IT)
(74) Representative: Martegani, Franco

(57) **Abstract**

Depilatory product characterized in that it comprises as base components, a glue and a solvent selected from vegetable oils.

## Description

The present invention relates to a depilatory product and the use of vegetable oils for depilatory products.

The depilatory products currently on the market and in particular depilatory waxes basically consist of two base components: a glue (generally the natural resin deriving from pine trees) and a solvent (oil) which give the product the necessary viscosity and stickiness, making it both fluid and at the same time easy to apply to the skin.

Furthermore, the presence of the solvent component (oil) is extremely important as it allows the product to be more easily removed and, above all, allows the removing tear to be more "elastic", i.e. less painful.

The solvent component (oil) used in the production of depilatory waxes currently on the market exclusively consists of vaseline oil. The use of vaseline oil is in fact particularly widespread in numerous food or pharmaceutical applications, as well as in the preparation of cosmetics. As it is a colourless and odourless product, vaseline oil does not alter the physical characteristics of the end-product.

The use of vaseline oil as solvent component in depilatory waxes, however, has various drawbacks.

In the first place, vaseline oil is a product of a mineral origin, as it is a derivative of petroleum, obtained by oil refining, and therefore suffers the effects in quantitative terms of the world production of crude oil and in terms of cost of the world quotation of crude oil.

Furthermore, the demand and request on the part of end-users of natural cosmetic products, i.e. based on substances of a natural origin (biological) are growing increasingly.

This demand has consequently led to the search for alternative products which, while guaranteeing the same or better functional characteristics of the end-product, at the same time provide a product marked by a natural formulation and high quality.

The Applicant has now surprisingly found that the particular product according to the present invention overcomes the drawbacks of the state of the art.

In particular, the product according to the present invention solves the technical problem of finding completely natural alternative products, not containing components deriving from petroleum.

An object of the present invention therefore relates to a depilatory product characterized in that it comprises as base components a glue and a solvent selected from vegetable oils.

In particular, the glue is a resin of a vegetable origin, preferably colophony, glyceril rosinate and/or mixtures thereof.

The solvent preferably consists of a vegetable oil selected from sunflower oil, palm oil, partially fractionated and refined vegetable oils, and/or mixtures thereof.

The depilatory product according to the present invention can comprise all the normal additives generally used for depilatory products.

A further object of the present invention relates to the use of vegetable oils as solvent component in depilatory products.

The depilatory product according to the present invention envisages that the glue be present in a quantity varying from 60 to 85% by weight with respect to the total weight of the depilatory product and preferably from 65 to 80% by weight with respect to the total weight of the depilatory product.

The depilatory product according to the present invention envisages that the solvent consisting of a vegetable oil be present in a quantity varying from 15 to 40% by weight with respect to the total weight of the depilatory product and preferably from 20 to 30% by weight with respect to the total weight of the depilatory product.

In the depilatory product according to the present invention, the glue is preferably colophony and the solvent is sunflower oil, or the glue is colophony and the solvent is bidistilled palm oil, or the glue is glyceril rosinate and the solvent is sunflower oil, or the glue is glyceril rosinate and the solvent is bidistilled palm oil, or the glue is a mixture of colophony and glyceril rosinate and the solvent is sunflower oil, or the glue is a mixture of colophony and glyceril rosinate and the solvent is bidistilled palm oil, or the glue is a mixture of colophony and glyceril rosinate and the solvent is a mixture of sunflower oil and bidistilled palm oil.

Consequently, a fundamental advantage of the product according to the present invention is that it consists of components of a natural origin only, in particular of a vegetable origin.

The depilatory product according to the present invention also has the considerable advantage of being a much less expensive product, and above all with a constant cost, as it does not depend on components which, deriving from petroleum, are subjected to the wavering cost and availability of petroleum and extremely high taxes.

A further advantage of the product according to the present invention is therefore that it consists of easily available components.

The depilatory product according to the present invention has even better characteristics with respect to the products according to the state of the art based on vaseline oil, especially in terms of "softness" of tear removal. It has in fact been possible to obtain a creamy depilatory product with a reduced stickiness but with a high depilatory power. Furthermore, when the skin is subjected to depilation with the depilatory product according to the present invention, it has a much lower reddening and dehydration level.

The depilatory product according to the present invention also has a viscosity equivalent to the product of the state of the art based on vaseline oil (measured according to the method ASTM D445), a perfect transparency and a typical resin odour, (this confirms that the vegetable oils used do not affect the smell). Furthermore from the spread tests effected, the depilatory product according to the present invention proves to be more fluid than the product according to the state of the art based on vaseline. It is therefore possible to keep the layer of the product constant during application on the test strip and then during the application of the product.

The depilatory product according to the present invention, comprising sunflower oil as solvent, provided a good result with respect to the physical and organoleptic properties and an excellent result as far as the depilatory effect is concerned.

The depilatory product according to the present invention, comprising a mixture of partially fractionated and refined vegetable oils as solvent, provided excellent results with respect to the physical and organoleptic properties and an excellent result as far as the depilatory effect is concerned, even better than the tests carried out with depilatory products based on vaseline oil of the state of the art.

Examples of depilatory products according to the present invention comprise the following components in the following quantity ranges:
a) colophony (70-80% by weight), sunflower oil (30-20% by weight);
b) colophony (65-75% by weight), glyceril rosinate (5-10% by weight), sunflower oil (10-30% by weight);
c) glyceril rosinate (70-80% by weight), sunflower oil (20-30% by weight);
d) colophony (70-80% by weight), bidistilled palm oil (30-20% by weight);
e) colophony (65-75% by weight), glyceril rosinate (5-10% by weight), bidistilled palm oil (30-20% by weight);
f) glyceril rosinate (70-80% by weight), bidistilled palm oil (20-30% by weight);
g) colophony (70-80% by weight), bidistilled palm oil (10-20% by weight), sunflower oil (10-20% by weight);
h) colophony (65-75% by weight), glyceril rosinate (5-10% by weight), bidistilled palm oil (10-20% by weight), sunflower oil (10-20% by weight);
i) glyceril rosinate (70-80% by weight), bidistilled palm oil (10-20% by weight), sunflower oil (10-20% by weight).
Tables 1 and 2 enclosed respectively illustrate the characteristics and composition of vegetable oils and sunflower oil.

**TABLE 1**

| (VEGETABLE OILS) | | |
|---|---|---|
| TECHNICAL SPECIFICATIONS | Product Code | AH10DA |
| PRODUCT: PREMIUM FRIT | Control date | 04/06/2004 |
| VEGETABLE PRODUCT FOR FRYING | Control Nr. | 0 |

### Description:

Vegetable product for frying. Obtained exclusively from a mixture of partially fractionated and refined vegetable oils for food use.

### Ingredients:

Sunflower-seed oil, fractionated palm oil, highly oleic sunflower-seed oil, antifoam agent (E900)

| **Physico-chemical characteristics** | **Method** | **Unit** | **Standard values** |
|---|---|---|---|
| Humidity | NGD C 3-1976 | % | max 0.10 |
| F.F.A (oleic acid) | NGD C10-1976 | % | max 0.10 |
| Peroxide number | NGD C35-1976 | meqO₂/kg | max 2.0 |
| Rancidity | NGD C56-1976 | | negative |
| Iodine number | NGD C32-1972 | gl₂/100g | 100-120 |
| Smoke point | NGD C77-1989 | °C | min 230 |

### Microbiological characteristics:

| | | |
|---|---|---|
| total bacterial charge | U.F.C./g | < 10 |
| mildew and yeast | U.F.C./g | < 10 |
| pathogens | | absent |

### Packaging:

The product is delivered in 10 litre cans

### Preservation

Preserve preferably in dry places far from heat sources.
**Shelf-life:** 12 (twelve) months.

**TABLE 2**

| (SUNFLOWER OIL) | | | | | |
|---|---|---|---|---|---|
| Oil type: Refined, loose sunflower oil | | | | | |
| Odour and taste: neutral, typical of sunflower oil, with no extraneous tastes. | | | | | |
| Characteristic | | Result | | | Method used |
| Adsorption at 420nm-453nm | | 0.06 | | 0.02 | 50/50 w/w in n-hexane |
| Colour Lovibond (R/Y 5" 1/4) | | N.A | | N.A. | ASTM 1544 - 68 |
| Acidity (% oleic acid) | | 0.04 | | % | NGD C10 - 1976 |
| Number of peroxides | | 1.16 | | meqO₂/kg | NGD C35 - 1976 |
| Soaps | | Abs. | | ppm | NGD C8 - 1976 |
| Cold test (24h at 0°C) | | Positive | | | NGD C24 - 1979 |
| Extraction solvent residues | | Inf. 1 | | ppm | A.O.C.S. Ca 3b -87 |
| Aromatic polycyclic hydrocarbons | | | | | |
| Benzo(a)Pyrene | | inf. 1 | | ppb | IUPAC - 1608 |
| Impurities | | < anal. limit % | | | NGD C7 - 1976 |

| Fatty Acid composition (Reg. CEE 2568/91 AII.XA - AII.XB on seed oil) | | | | | |
|---|---|---|---|---|---|
| Results | | | Reference values (ISO 5508 NGD Ed 2002 | | |
| Myristic acid | 0.07 | | Max 0.2% | | |
| Palmitic acid | 6.40 | | 5%<Palmitic acid <7.6% | | |
| Stearic acid | 3.29 | | 2.7%< Stearic acid<6.5% | | |
| Oleic acid | 32.63 | | 14%<Oleic acid<39.4% | | |
| Linoleic acid | 56.08 | | 48.3%<Linoleic acid<74% | | |
| Linolenic acid | 0.23 | | Max. 0.3% | | |
| Arachidic acid | 0.22 | | 0.1 %<Arachidic acid<0.5% | | |
| Elcosenic acid | 0.15 | | Max. 0.3% | | |
| Beenic acid | 0.58 | | 0.3%<Beenic acid<1.5% | | |
| Lignoceric acid | 0.11 | | Max.0.5% | | |

The values shown refer to the analysis effected on the oil contained in the feeding tank

## Claims

1. A depilatory product **characterized in that** it comprises a glue and a solvent selected from vegetable oils, as base components.

2. The depilatory product according to claim 1, **characterized in that** the glue is a resin of a vegetable origin.

3. The depilatory product according to claim 2, **characterized in that** the glue is selected from colophony, glyceril rosinate and/or mixtures thereof.

4. The depilatory product according to claim 1, **characterized in that** the solvent consists of a vegetable oil selected from sunflower oil, palm oil, partially fractionated and refined vegetable oils, and/or mixtures thereof.

5. The depilatory product according to claim 1, **characterized in that** it comprises normal additives used for depilatory products.

6. The depilatory product according to claim 1, **characterized in that** the glue is present in a quantity varying from 60 to 85% by weight with respect to the total weight of the depilatory product.

7. The depilatory product according to claim 1, **characterized in that** the glue is present in a quantity varying from 65 to 80% by weight with respect to the total weight of the depilatory product.

8. The depilatory product according to claim 1, **characterized in that** the vegetable oil is present in a quantity varying from 15 to 40% by weight with respect to the total weight of the depilatory product.

9. The depilatory product according to claim 1, **characterized in that** the vegetable oil is present in a quantity varying from 20 to 30% by weight with respect to the total weight of the depilatory product.

10. The depilatory product according to claim 1, **characterized in that** the glue is colophony, glyceril rosinate and/or mixtures thereof and the solvent is selected from sunflower oil, palm oil, seed oils and/or mixtures thereof.

11. The depilatory product according to claim 1, **characterized in that** the glue is colophony and the solvent is sunflower oil.

12. The depilatory product according to claim 1, **characterized in that** the glue is colophony and the solvent is bidistilled palm oil.

13. The depilatory product according to claim 1, **characterized in that** the glue is glyceril rosinate and the solvent is sunflower oil.

14. The depilatory product according to claim 1, **characterized in that** the glue is glyceril rosinate and the solvent is bidistilled palm oil.

15. The depilatory product according to claim 1, **characterized in that** the glue is a mixture of colophony and glyceril rosinate and the solvent is sunflower oil.

16. The depilatory product according to claim 1, **characterized in that** the glue is a mixture of colophony and glyceril rosinate and the solvent is bidistilled palm oil.

17. The depilatory product according to claim 1, **characterized in that** the glue is a mixture of colophony and glyceril rosinate and the solvent is a mixture of sunflower oil and bidistilled palm oil.

18. Use of vegetable oils as solvent component in depilatory products.

19. Use of vegetable oils as solvent component in depilatory products according to any of the claims from 1 to 17.
